# EUROPEAN PATENT APPLICATION

(11) **EP 0 687 710 A2**
(43) Date of publication of application: **20.12.1995**
(21) Application number: 95303430.3
(22) Date of filing: 23.05.1995
(51) Int. Cl.: C08L 69/00, C08L 67/02, A61L 2/08

(54) **Copolyester-carbonate resin/PCT blends**

(30) Priority: 14.06.1994 US 259589
(71) Applicant: GENERAL ELECTRIC COMPANY, Schenectady, NY 12345 (US)
(72) Inventor: DeRudder, James Louis, Mount Vernon, Indiana 47620 (US)
(74) Representative: Szary, Anne Catherine

(57) **Abstract**

A thermoplastic molding composition, a molded article of improved color, and method of preparing a thermally molded, sterile article of improved color, which includes: (a) formulating a thermoplastic molding composition including from about 50 to about 99% by weight of at least one copolyester-carbonate resin; and from about 1 to about 50% by weight of at least one polyester resin which is a condensation product of at least one diacid and at least one polyol in which the at least one diacid is comprised of greater than about 50% by weight of terephthalic acid and the at least one polyol is comprised of greater than about 50% by weight of 1,4-cyclohexanedimethanol, which is one of a homopolymer, a copolymer, and mixtures thereof, and which is blended at a temperature effective to at least melt the at least one polyester resin and provide a blend which has a single phase; (b) molding an article in a solid form from the thermoplastic molding composition; (c) sealing the article in a moisture-proof, microorganism-impermeable, ionizing ray-permeable container; and (d) subjecting the sealed-in article to a non-destructive, sterilizing dose of an ionizing ray.

## Description

### 1. FIELD OF THE INVENTION

This invention relates to an article of improved color molded from a thermoplastic molding composition and sterilized by ionizing radiation, and, more particularly, to such an article of improved color molded from a thermoplastic molding composition which is a melt blend of at least one copolyester-carbonate resin and at least one polyester resin which is a PCT-based resin, i.e., a poly(1,4-cyclohexanedimethylene terepthalate)based resin.

### 2. BACKGROUND OF THE RELATED ART

Synthetic thermoplastic polymeric resins have been used increasingly to mold articles useful in medicine and surgery. Examples of such articles include containers, packaging, instruments, prosthetics, tubing, and working components of treatment apparatus.

The selection of a synthetic thermoplastic polymeric resin for a particular type of molded article depends on the physical properties required in the molded article. One property necessary for many medical and surgical articles molded from thermoplastic polymeric resins is receptivity to sterilization procedures.

A commonly preferred sterilization technique is exposure to ionizing radiation. Unfortunately, ionizing radiation may adversely impact some polymeric resins in ways unacceptable for some uses. For example, polycarbonate resins have many properties which are advantageous to their use in many medical and surgical devices or articles. Upon exposure to ionizing radiation, however, articles molded from polycarbonate resins change from a normal transparency and clarity to a yellowed coloration with reduced transparency. In addition to being less acceptable aesthetically, these coloration and transparency changes are unstable, and change further even after gamma ray exposure.

A number of compounds have been found to inhibit the yellowing of articles molded from polycarbonate resins when subjected to ionizing radiation. Representative of these yellowing inhibitors are those described in U.S. Patent Nos. 4,624,972 (Nace); 4,657,949 (Nace); 4,757,104 (Nace); and 4,804,692 (Lundy et al.). The presence of any additive in a polycarbonate resin molding composition, however, generally has an effect on other desirable physical properties of articles molded therefrom.

Another strategy for reducing the yellowing of articles molded from polycarbonate resins upon exposure to ionizing radiation is described in U.S. Patent No. 4,778,656 (Allen et al.). The method entails blending the polycarbonate resin with another polymer, which has the effect of improving ionizing radiation resistance of articles molded therefrom compared to that of articles molded from the polycarbonate alone. Examples of the additive polymers include polyesters, polysulfone-carbonates and certain copolyesters. Among the polymers which are described as employable in blends with polycarbonate are 1,4-cyclohexanedimethanol-containing polyesters.

Table 1 of U.S. Patent No. 4,778,656 gives specific examples of blends of polycarbonate with a copolyester of terephthalic acid and about 80 mole % 1,4-cyclohexanedimethanol and about 20 mole % ethylene glycol (KODAR PCTG, Eastman Chemical Company), and a copolyester of 1,4-cyclohexanedimethanol, about 85 wt. % terephthalic acid and about 15 wt. % isophthalic acid (KODAR A150, Eastman Chemical Company). The maximum reduction in yellowing for blends including the copolyester containing the diol comonomer ethylene glycol occurred for blends including from 60-80 wt. % of the copolyester. The maximum reduction in yellowing for the blend including the copolyester containing acid portions appears to occur for blends including from 40-50 wt. % of the copolyester. Like the previously described yellowing inhibitors, these additive polymers also effect the physical properties of articles molded from blends of these polymers.

My prior U.S. Patent No. 5,137,688 discloses thermoplastic articles molded from a blend of an amorphous polyamide resin and a polycarbonate resin which exhibit improved color when irradiated with ionizing radiation. Like the previously described yellowing inhibitors and additive polymers, additive amorphous polyamide resins also effect the physical properties of articles molded from blends of the amorphous polyamide resin and a polycarbonate resin.

A further attempt to blend polycarbonates with various polymeric systems is described in DE 16 94 124 wherein from 1 to 5 wt. % polyalkylene terephthalates are employed. Among the polyalkylene terephthalates specifically exemplified is poly(1,4-cyclohexanedimethanol terephthalate).

Also of interest are Kawase and Grundmeier U.S. Patent Nos. 3,953,539 and 4,056,504, respectively.

U.S. Patent No. 4,391,954 to Scott discloses thermoplastic molding compositions containing a 1,4-cyclohexanedimethanol-containing polyesters melt blended with a polycarbonate. Compositions containing from 1 to 99 parts by weight polycarbonate and from 99 to 1 parts by weight of a polyester derived from cyclohexanedimethanol and a mixture of isophthalic and terephthalic acids are disclosed. Preferably, the compositions contain from 2 to 75 parts by weight of this polyester. The polycarbonate is said to be compatible with this polyester over a wide range, while the molded article maintains the transparency characteristic of the polycarbonate.

U.S. Patent No. 4,786,692 to Allen et al. discloses blends of polycarbonate with a polyester containing 1,4-cyclohexanedimethanol and ethylene glycol in 1:4 molar quantities. The blends are said to exhibit lower heat distortion temperatures while retaining good tensile, flexural and impact strength.

Eastman Chemical Company's Laid-Open Patent Application PCT 09 911, published July 7th, 1991A discloses a composition including from 10-90 wt. % polycarbonate and from 10-90 wt. % 1,4-cyclohexane dimethanol terephthalate, wherein from 1-50 wt. % of these two polymers are transesterified to form a complex copolymer. The composition is useful for medical objects.

An article by Mohn et al. appearing in the Journal of Applied Polymer Science, Vol.23 (1979), pps. 575-587, discloses a crystalline non-transparent blend of 75 wt. % aromatic polycarbonate and 25 wt. % 1,4-cyclohexanedimethanol terephthalate.

Several patents have issued recently to Fontana et al. which describe a new class of copolyester-carbonate resins, see, for example, U.S. Patent Nos. 4,983,706: 5,025,081: and 5,106,904, the disclosures of which are incorporated herein by reference. This new class of resins is prepared by a process which comprises reacting interfacially at least one dihydric phenol, at least one carbonate precursor, and at least one aliphatic alpha omega dicarboxylic acid or ester having from about nine to about 40 carbon atoms and being, preferably, saturated, wherein the dicarboxylic acid or ester precursor is present in the copolyester-carbonate resin in quantities ranging from about two to about 30 mole % based on the dihydric phenol.

Thus, while it is known to improve the color upon irradiation with ionizing radiation of articles molded from polycarbonate resins blended with select yellowing inhibitors and select additive polymers, no truly satisfactory blend has been obtained as yet.

It is therefore an object of the present invention to provide a thermoplastic molding composition, which is a novel blend based on at least one copolyester-carbonate resin.

It is a further object of the present invention to provide articles molded from the foregoing thermoplastic molding composition which have a good optical transparency and an improved irradiation resistance compared to that of articles molded from the at least one copolyester-carbonate resin alone.

It is yet another object of the present invention to provide a method of preparing a thermally molded, sterile article of improved color from the foregoing thermoplastic molding composition.

### SUMMARY OF THE INVENTION

These and other objects are achieved by the present invention which provides a thermoplastic molding composition which is a blend, comprising: from about 50 to about 99 percent by weight of at least one copolyester-carbonate resin; and from about 1 to about 50% by weight of at least one polyester resin which is a condensation product of at least one diacid and at least one polyol in which the at least one diacid is comprised of greater than about 50% by weight of terephthalic acid and the at least one polyol is comprised of greater than about 50% by weight of 1,4-cyclohexanedimethanol, and which is one of a homopolymer, a copolymer, and mixtures thereof. Preferably, the at least one polyester resin is a poly(1,4-cyclohexanedimethylene terephthalate).

The present invention additionally provides an article of improved color, comprising an article which has been subjected to sterilization by ionizing radiation and which has been molded from a thermoplastic molding composition as described above. Such a thermally molded, sterile article of improved color is provided by a method, comprising: a. formulating a thermoplastic molding composition which comprises the composition described above and which is blended at a temperature effective to at least melt the at least one polyester resin and provide a melt blend which has a single phase; b. molding an article in a solid form from the thermoplastic molding composition; c. sealing the article in a moisture-proof, microorganism-impermeable, ionizing ray-permeable container: and d. subjecting the sealed-in article to a non-destructive, sterilizing dose of an ionizing ray.

### DETAILED DESCRIPTION OF THE PREFERRED EMBODIMENTS

The copolyester-carbonate resins useful in this invention include copolyester-carbonate resins such as those disclosed by Fontana et al. in U.S. Patent Nos. 4,983,706; 5,025,081; and 5,106,904, the disclosures of which are herein incorporated by reference. These copolyestercarbonate resins are a new class of resins prepared by a process which comprises reacting interfacially at least one dihydric phenol, at least one carbonate precursor, and at least one aliphatic alpha omega dicarboxylic acid or ester having from about nine to about 40 carbon atoms and being, preferably, saturated, wherein the dicarboxylic acid or ester precursor is present in the copolyester-carbonate resin in quantities ranging from about 2 to about 30 mole % based on the dihydric phenol.

The copolyester-carbonate resin which is employed in the composition of the invention is also referred to as a "soft segment polycarbonate". This refers to the fact that the material is a block copolymer having a rigid polycarbonate segment and a soft segment which is less rigid and which depresses the glass transition temperature to provide better impact resistance for the block copolymer. The soft segment may be, for example, an acid end capped aliphatic having about 6 carbon atoms, i.e., adipic acid, or about 12 carbon atoms, i.e., an aliphatic diacid, such as dodecane dioic acid.

The preferred copolyester-carbonate resin employed in the present invention is a soft segment polycarbonate prepared according to the interfacial copolymerization reaction of the previously mentioned U.S. Patents to Fontana et al. in which about 10 wt. % of an aliphatic diacid having 12 carbon atoms is included, i.e., dodecane dioic acid.

The polyester resin which is employed in the composition of this invention is a condensation product of at least one diacid and at least one polyol, preferable a diol. The at least one diacid is comprised of greater than about 50% by weight of terephthalic acid and the at least one polyol is comprised of greater than about 50% by weight of 1,4-cyclohexanedimethanol (CHDM). Preferably, the at least one diacid is comprised of greater than about 75% by weight of terephthalic acid and the at least one polyol is comprised of greater than about 75% by weight of 1,4-cyclohexanedimethanol (CHDM). The CHDM may have a cis configuration, a trans configuration, or a mixture thereof.

The composition may thus contain at least one polyester which is one of a homopolymer, a copolymer, and mixtures thereof. The preferred polyester is a homopolymer and is a poly(1,4-cyclohexanedimethylene terephthalate), which is known in the art as a PCT.

Such PCT homopolymers are commercially available from Eastman Chemical Company as PCT-3879. The inherent viscosity is about 0.770 dl/gm, and ranges from a minimum of about 0.74 to a maximum of about 0.800 dl/gm. When procuring this material from Eastman Chemical Company, it is preferable to specify a melting point peak of about 295.0°C, with a minimum of about 291.0 and a maximum of about 299.0°C. It is also preferable to obtain material having a known recrystallization temperature peak because the properties of this polyester vary with the cis/trans ratio of the 1,4-cyclohexanedimethanol. While a polyester having any cis/trans ratio may be employed for the compositions of the present invention, it is helpful to know the melting point peak and the recrystallization temperature in order to obtain a true melt blend of the polyester and the copolyester-polycarbonate resin. The melting point is an indirect measure of the cis-trans ratio, since melting point increases with trans content.

Eastman Chemical Company's PCT-3879 is thus a member of a group of 1,4-cyclohexanedimethanol-based polyesters in which the 1,4-cyclohexane dimethanol constitutes all or part of the diol (glycol) employed to prepare the polyester. While the PCT-3879 is a polyester of 1,4-cyclohexanemethanol and terephthalic acid, Eastman Chemical Company markets other members of the 1,4-cyclohexanedimethanol-based polyester group. These additional materials include KODAR A150, which is a copolyester of 1,4-cyclohexanedimethanol and about 85 wt. % terephthalic acid and about 15 wt. % isophthalic acid, and KODAR PCTG, which is a polyester of terephthalic acid and about 80 mole % 1,4-cyclohexanedimethanol, and about 20 mole % ethylene glycol. These copolyester members of the CHDM-based polyester group are additionally useful for the present invention, alone, as mixtures of copolyesters, and/or as mixtures with PCT homopolymers.

The copolyesters of the present invention are PCT-based copolyesters and are condensation products of at least one diacid and at least one polyol in which the at least one diacid is comprised of greater than about 50% by weight of terephthalic acid and the at least one polyol is comprised of greater than about 50% by weight of 1,4-cyclohexanedimethanol. Suitable diacids include, but are not limited to, in the order of preference, aromatic diacids, such as isophthalic acid, alicyclic diacids, such as cyclohexane dicarboxylic acid, and complex aliphatic diacids. Suitable polyols include, but are not limited to diols, such as ethylene glycol, propylene glycol, and butylene glycol, and various triols employed at low levels, for example, about 2% by weight, so as to minimize undesirable branching.

The composition of the present invention is a true blend of two types of polymers, (A) at least one copolyester-carbonate resin and (B) at least one polyester resin as defined herein to include homopolymers, copolymers and mixtures thereof. Preparation of admixtures of the polymers may be carried out in any conventional manner. In the usual case, pellets or finely divided dry powders of the polymers are simply blended together in a mechanical mixer and the blend is compounded by passage through an extruder at an elevated temperature above the softening points and melting points of the polymeric constituents. This method is the preferred method for the present invention because it is desirable to have a true blend, i.e., a blend of miscible polymers, and this is possible when the polyester is melt blended at above about 300°C, that is, above its melting point peak of approximately 295.0°C so as to provide a melt blend having a single phase. While the order of addition of materials is not important, it is necessary to melt the crystalline polyester in order to get a transparent blend of miscible polymers having a single phase. For the soft-segment polycarbonates employed in the present invention, inclusion of aliphatic diacids as the soft segment produces improved ductility for the polymer chain, improved impact resistance and flow properties.

Admixtures may alternatively be prepared by other techniques, such as, dissolving the polymers in a mutual solvent from which they are subsequently recovered in blended form. Recovery may be by evaporation, distillation, or precipitation as is known in the art.

Blends of the present invention are particularly useful because their composition may be varied to provide continuous variation of the thermal characteristics of the resultant blends. Many biological systems and devices for use with biological systems have very well defined upper temperature limits. The present invention, therefor allows the thermal capability of the blend to be modified to match the requirements of a system or device while maintaining the desirable physical properties of a bisphenol A polycarbonate.

The resulting polymer blend can be further modified if desired, by incorporation of standard amounts of conventional additives, such as thermal stabilizers, for example, a phosphite: an anti-oxidant, for example, a hindered phenol; as well as any of the well-known mold release agents; colorants; fillers; and the like.

The blends of the present invention can be used for various applications, including compact disks, automotive applications, and in the medical field. However, blends according to the present invention are particularly well suited for molding transparent articles useful in the medical field which must be sterilized prior to use. Polycarbonates have previously been a suggested material of choice for such articles and the present invention now teaches that a new class of resins, copolyester-carbonate resins, provide thermoplastic molding compositions based thereon having a spectrum of properties making them useful for molding transparent articles to be used in the medical field which must be sterilized prior to use. Like polycarbonates, copolyester-carbonate resins yellow during and/or after irradiation with a sterilizing dose of ionizing rays, such as gamma rays. There is thus the need for formulations based on these copolyester-carbonates which have improved irradiation resistance.

The terms "sterile" and "sterilizing" as used herein mean the absence (or killing) of undesirable microorganisms within the limits prescribed by the United States Pharmacopie XXII (1990). This definition, it is to be noted, is not in accordance with the classical definition formulated by the Council on Pharmacy and Chemistry of the American Medical Association. The methods of determining sterility and the specification for sterility may be in accordance with the U.S. Pharmacopie XXII; (see 71, pages 1483-1488).

The term "ionizing-ray" as used herein means ionizing radiation. The term "ionizing radiation" means radiation possessing an energy possessing an energy at least sufficient to produce ions or to break chemical bonds and thus includes radiation, such as "ionizing particle radiation", as well as radiation of the type termed "ionizing electromagnetic radiation".

The term "ionizing particle radiation" is used to designate the emission of electrons or highly accelerated, relatively heavy, nuclear particles, such as protons, neutrons, alpha particles, deuterons, beta particles, or their analogs directed in such a way that the particle is projected into the mass to be irradiated. Charged particles can be accelerated by voltage gradients provided by devices, such as accelerators with resonance chambers, Van der Graaff generators, insulating core transformers, betatrons, synchrotrons, cyclotrons, and the like. Neutron radiation can be produced by bombarding a selected light metal, such as beryllium, with positive particles of high energy. Particle radiation can also be obtained by the use of an atomic pile, radioactive isotopes, or other natural or synthetic radioactive materials.

"Ionizing electromagnetic radiation" is produced when a metallic target, such as tungsten, is bombarded with electrons of suitable energy. This energy is conferred to the electrons by potential accelerators over 10,000 electron volts. In addition to radiation of this type, commonly called X-ray, an ionizing electromagnetic radiation suitable for the practice of this invention may be obtained by means of a nuclear reactor (a pile) or by the use of natural or synthetic radioactive material, for example, cobalt 60. The use of cobalt 60 as a source of ionizing radiation, producing gamma rays, is preferred in the method of the present invention.

An unexpected and significantly improved irradiation resistance has been found for thermoplastic molding compositions according to the invention which are blends of from about 50 to about 99 % by weight of at least one copolyestercarbonate resin and from about 1 to about 50% by weight of at least one polyester resin which is a poly(1,4-cyclohexanedimethylene terephthalate). Preferably the composition is a blend of from about 75 to about 90% by weight of the at least one copolyester-carbonate resin, and from about 10 to about 25% by weight of the at least one polyester resin. Most preferably the composition comprises about 90% by weight of the at least one copolyester-carbonate resin and about 10% by weight of the at least one polyester resin. The blend is preferably a melt blend having a single phase which has a good optical transparency and an improved irradiation resistance compared to that of the at least one copolyester-carbonate resin alone.

Articles may be formed by any of several well-known techniques, for example, extrusion, injection molding, and blow molding. It is critical, however, that sufficient time at molding temperature is provided so that any crystallizable or crystalline polyester component can be thoroughly melted into an amorphous state and then the amorphous polyester melt and the amorphous polycarbonate melt can be melt mixed. Articles according to the present invention have improved color after being subjected to sterilization by ionizing radiation as defined herein compared to articles of copolyester-carbonate resin alone.

The present invention includes a method of preparing a thermally molded, sterile article. The method includes providing an article molded in a solid form from a thermoplastic molding composition which is a blend according to the invention as discussed in the foregoing; sealing the article in a moisture-proof microorganism-impermeable, ionizing ray-permeable container, and subjecting the sealed-in article to a non-destructive, sterilizing dose of an ionizing ray.

The initially provided molded articles are hermetically sealed in a moisture-proof, micro-organism-impermeable, ionizing ray-permeable container. Preferably, the articles are sealed in pouches, multiple containers, such as overwraps or similar containers made of non-metallic materials which will effectively exclude infiltration of microorganisms, gas, vapor and moisture over a time period of several years. Such packaging materials are commercially available in numerous forms of polymeric films, including laminates of two or more films. For example, the pouches may be constructed of polyethylene, polypropylene, polyethyleneterephthalate, polyvinyl chloride, and like polymeric films for forming hermetically sealed pouches. It will be appreciated that the container should be initially provided in clean, particulate-free condition and that they be presterilized to some extent employing conventional techniques, such as ultraviolet radiation and the like.

After the molded articles are sealed in the above-described containers, they are subjected, according to the method of the invention, to a non-destructive (non-degrading), sterilizing dose of an ionizing ray as defined in the foregoing.

A non-degrading, sterilizing dose of ionizing radiation for the articles of the invention is advantageously within the range of from about 0.5 to 10.0 megarads; preferably not more than 4.0 megarads. Radiation within this dosage range may be carried out at room temperature or below or at elevated temperatures if so desired. The temperature at which the radiation is carried out is not critical to the method of the invention. However, practical temperatures are within the range of from about -10°C to about 50°C. Lower radiation dosages may not be effective to sterilize the sealed-in articles. Higher doses will generally degrade (destroy) either the package or the article contained therein or both. This, of course, is undesirable. For this reason, preferably the dosage employed for sterilizing the sealed articles is within the range of from about 1 to about 3 megarads, most preferably about 2.5 megarads.

As mentioned in the foregoing, gamma radiation produced by cobalt 60 is a preferred ionizing ray for employment in the method of the invention. Alternative sources of ionizing radiation, such as electron beam and x-ray, can also be utilized. Gamma radiation produced by cobalt 60 has a high penetrating ability and obviates the need for concern about the thickness of the article to be penetrated. It is well-known that microorganisms exposed to radiation, including gamma radiation, do not always die immediately. In some bacteria, which have been subjected to radiation dose which prevents their multiplication, many biological functions continue for extended periods of time. For this reason, sterility testing to be carried out as a control mechanism should be delayed for a period of about four days following radiation.

Apparatus for producing ionizing rays and techniques for their application to a wide variety of materials are so well-known that further description need not be given herein. Those skilled in the art will appreciate the techniques of ionizing ray application.

The following examples describe the manner and process of making and using the invention, and set forth the best mode contemplated by the inventor for carrying out the invention. These examples, however, are not to be construed as limiting the invention.

### Examples

Blends of a copolyester-carbonate and a polyester resin were prepared as follows. The copolyester-carbonate resin employed was a soft segment polycarbonate which is a block copolymer of a polycarbonate and an aliphatic diacid having 12 carbon atoms (LEXAN SP, General Electric Company, Mount Vernon, Indiana) and the polyester resin employed was a poly(1,4-cyclohexanedimethylene terephthalate) (Eastman Chemical Company's PCT-3879 which was specified to have a melting point peak of about 295.0°C, with a minimum of about 291.0 and a maximum of 299.0°C, and an inherent viscosity of about 0.770 dl/gm, with a minimum of about 0.740 and a maximum of 0.800 dl/gm). The blends were compounded on a Werner Pfleiderer ZSK 30 mm twin screw extruder at a temperature of at least 300°C. The resulting pellets were dried for at least six hours at 110°C before injection molding into ASTM test specimens (chips) on an 80 ton, four oz. injection molding machine. The chips had a thickness of 0.125 inches and were irradiated with various doses of ionizing rays from a cobalt 60 source and then tested for yellowness index at various times. Yellowness index (YI) was measured according to ASTM test method D-1925 both before irradiation and after irradiation. Compositions according to the invention (Samples B-E) are set forth in Table 1 as is a composition for comparative purposes (Sample A).

Samples irradiated with gamma radiation of 2.5 and 5.0 megarads were initially measured for changes in yellowness index (dYI)¹ one day after irradiation. Samples irradiated with 7.5 megarads were initially measured for yellowness index four days after irradiation. In both cases, the samples were not exposed to light until the initial color change measurement was made. After initial radiation, the samples were aged while exposed to ambient lighting conditions (about 75 footcandles, fluorescent light fixtures) and a second yellowness index determination (dYI)² made one week after the initial gamma irradiation. The results are set forth in Table 1.

**Table 1**

| | SSPC/PCT | EXPOSURE | INITIAL COLOR CHANGE AFTER GAMMA IRRADIATION | COLOR CHANGE ONE WEEK AFTER GAMMA IRRADIATION |
|---|---|---|---|---|
| SAMPLE | (wt. %) | (Megarad) | (dYI)¹ | (dYI)² |
| A | 100/0 | 2.5 | 25.9 | 6.3 |
| B | 95/5 | 2.5 | 17.3 | 3.7 |
| C | 90/10 | 2.5 | 11.5 | 2.6 |
| D | 75/25 | 2.5 | 10.5 | 1.0 |
| E | 50/50 | 2.5 | 6.7 | 2.6 |
| A | 100/0 | 5.0 | 52.6 | 31.6 |
| B | 95/5 | 5.0 | 41.6 | 20.0 |
| C | 90/10 | 5.0 | 32.5 | 13.5 |
| D | 75/25 | 5.0 | 29.1 | 10.1 |
| E | 50/50 | 5.0 | 24.7 | 17.4 |
| A | 100/0 | 7.5 | 54.1 | 43.0 |
| B | 95/5 | 7.5 | 41.6 | 30.3 |
| C | 90/10 | 7.5 | 34.1 | 24.0 |
| D | 75/25 | 7.5 | 29.3 | 20.5 |
| E | 50/50 | 7.5 | 24.2 | 15.8 |

As can be seen from the data, the copolyester-carbonate resin (Sample A) turns yellow (increasing YI) with gamma ray irradiation. Blends of the copolyester-carbonate with PCT have significantly improved resistance to yellowing after exposure to gamma ray irradiation. optimal blends appear to be those in which the PCT ranges from about 10 to about 25 wt. %. Blends containing about 50% PCT show improved resistance to yellowing in the initial color change measurement, but show poorer performance after being aged in ambient light when the color change was measured one week after gamma irradiation. This effect was less with 7.5 megarad exposure, but appears to be significant for 2.5 and 5.0 megarad exposure.

It is understood that various other modifications will be apparent to and can be readily made by those skilled in the art without departing from the scope and spirit of the present invention. Accordingly, it is not intended that the scope of the claims appended hereto be limited to the description set forth above but rather that the claims be construed as encompassing all of the features of patentable novelty which reside in the present invention, including all features which would be treated as equivalents thereof by those skilled in the art to which the invention pertains.

## Claims

1. A thermoplastic molding composition which is a blend, comprising:
from about 50 to about 99% by weight of at least one copolyester-carbonate resin; and
from about 1 to about 50% by weight of at least one polyester resin which is a condensation product of at least one diacid and at least one polyol in which the at least one diacid is comprised of greater than about 50% by weight of terephthalic acid and the at least one polyol is comprised of greater than about 50% by weight of 1,4-cyclohexanedimethanol, and which is one of a homopolymer, a copolymer, and mixtures thereof.

2. The thermoplastic molding composition according to claim 1, wherein the composition comprises from about 75 to about 95% by weight of the at least one copolyester-carbonate resin, and from about 5 to about 25% by weight of the at least one polyester resin.

3. The thermoplastic molding composition according to claim 1, wherein the at least one copolyester-carbonate resin is a reaction product of at least one dihydric phenol, at least one carbonate precursor, and at least one aliphatic alpha omega dicarboxylic acid or ester precursor thereof, wherein the at least one aliphatic alpha omega dicarboxylic acid or ester precursor has from about 9 to about 40 carbon atoms and is present in the copolyester-carbonate in quantities ranging from about 2 to about 30 mole % based on the dihydric phenol.

4. The thermoplastic molding composition according to claim 3, wherein the at least one aliphatic alpha omega dicarboxylic acid or ester precursor thereof includes at least one saturated aliphatic alpha omega dicarboxylic acid or ester precursor thereof.

5. The thermoplastic molding composition according to claim 3, wherein the at least one aliphatic alpha omega dicarboxylic acid or ester precursor thereof is present in the amount of about 10 mole % based on the dihydric phenol.

6. The thermoplastic molding composition according to claim 1, wherein the at least one copolyester-carbonate resin and the at least one polyester resin are melt blended at a temperature effective to at least melt the at least one polyester resin and provide a blend which has a single phase.

7. The thermoplastic molding composition according to claim 6, wherein the temperature is at least about 300°C.

8. The thermoplastic molding composition according to claim 6, wherein the blend has a good optical transparency and an improved irradiation resistance compared to that of the at least one copolyester-carbonate resin alone.

9. An article of improved color, comprising:
an article which has been subjected to sterilization by ionizing radiation and which has been molded from a thermoplastic molding composition which is a blend comprised of:
from about 50 to about 99% by weight of at least one copolyester-carbonate resin; and
from about 1 to about 50% by weight of at least one polyester resin which is a condensation product of at least one diacid and at least one polyol in which the at least one diacid is comprised of greater than about 50% by weight of terephthalic acid and the at least one polyol is comprised of greater than about 50% by weight of 1,4-cyclohexanedimethanol, and which is one of a homopolymer, a copolymer, and mixtures thereof.

10. A method of preparing a thermally molded, sterile article of improved color, comprising:
a. formulating a thermoplastic molding composition which comprises:
i. from about 50 to about 99% by weight of at least one copolyester-carbonate resin; and
ii. from about 1 to about 50% by weight of at least one polyester resin which is a condensation product of at least one diacid and at least one polyol in which the at least one diacid is comprised of greater than about 50% by weight of terephthalic acid and the at least one polyol is comprised of greater than about 50% by weight of 1,4-cyclohexanedimethanol, and which is one of a homopolymer, a copolymer, and mixtures thereof, and which is melt blended at a temperature effective to at least melt the at least one polyester resin and provide a melt blend which has a single phase;
b. molding an article in a solid form from the thermoplastic molding composition;
c. sealing the article in a moisture-proof, microorganism-impermeable, ionizing ray-permeable container; and
d. subjecting the sealed-in article to a non-destructive, sterilizing dose of an ionizing ray.
